# EUROPEAN PATENT SPECIFICATION

(11) **EP 1 066 854 B1**
(45) Date of publication and mention of the grant of the patent: **19.09.2007**
(21) Application number: 00305463.2
(22) Date of filing: 29.06.2000
(51) Int. Cl.: A61P 33/10, A61K 31/415, A61K 31/365

(54) **Anthelmintic compositions containing combinations of avermectins or milbemycins with bis-aryl compounds**
Avermectinen oder Milbemycine und bis-Aryl-Derivate enthaltende Anthelminthische Zusammensetzungen
Compositions anthelminthiques contenant un dérivé d'avermectin ou de milbemycin et un composé bis-aryle

(30) Priority: 08.07.1999 GB 9916052
(43) Date of publication of application: 10.01.2001
(60) Divisional application: 07111879.8
(73) Proprietor: PFIZER INC., New York, N.Y. 10017 (US); Pfizer Limited, Sandwich, Kent CT13 9NJ (GB)
(72) Inventor: Bruce, Christopher Ian, Sandwich, Kent CT13 9NJ (GB)
(74) Representative: Stuart, Peter Graham

(56) References cited:
- WO-A-98/11780
- CUTLER S L: "Ectopic Psoroptes cuniculi infestation in a pet rabbit." JOURNAL OF SMALL ANIMAL PRACTICE, (1998 FEB) 39 (2) 86-7. , XP001128780

## Description

This invention relates to combinations, especially synergistic combinations of an avermectin or a milbemycin which is selectamin with a bis-aryl compounds which is fipronil and their use in combating parasite, especially helminth, infections.

The avermectins are a group of broad spectrum antiparasitic agents referred to previously as the C-076 compounds. They are produced by fermenting a strain of the micro-organism Streptomyces avermitilis under aerobic conditions in an aqueous nutrient medium containing inorganic salts and assimilable sources of carbon and nitrogen. The isolation and the chemical structure of the eight individual components which make up the C-076 complex is described in detail in British Patent Specification 1573955.

The C-076 complex comprises eight distinct but closely related compounds described as C-076 A1a, A1b, A2a, A2b, B1a, B1b, B2a and B2b. The "a" series of compounds refers to the natural avermectins wherein the 25-substituent is (S)-sec-butyl and the "b" series to those wherein the 25-substituent is isopropyl. The designations "A" and "B" refer to avermectins wherein the 5-substituent is methoxy or hydroxy, respectively, and the numeral "1" refers to avermectins wherein a double bond is present at the 22-23 position, and numeral "2" to avermectins wherein there is a single bond between C-22 and C-23 and wherein there is a 23-hydroxy substituent.

In our European Patent Applications 0214731, 0284176, 0317148, 0308145, 0340832, 0335541 and 0350187 there are described preparations of compounds related to the naturally-occurring avermectins but having a group at the 25-position other than the isopropyl or (S)-sec-butyl groups found in the original avermectin compounds disclosed in British Patent Specification 1573955. Such compounds may be prepared by fermentation of particular strains of Streptomyces avermitilis in the presence of organic acids or derivatives thereof. Production of such avermectins is described in Journal of Antibiotics (1991), 44, No. 3, pp 357-365.

The milbemycins form another group of related macrolides which are distinguished from the avermectins in lacking a sugar residue attached at the C-13 position. Examples of such compounds are described in UK patent 1390336, and European patent publications 170006, 254583, 334484 and 410615. In addition to these fermentation products a large number of publications describe compounds derived semisynthetically from them, many of which possess useful antiparasitic activities. Some of this chemistry is reviewed in Macrolide Antibiotics, Omura S., Ed., Academic press, New York (1984) and by Davies, H.G., Green, R.H. in Natural product Reports (1986), 3, 87-121 and in Chem. Soc. Rev., 1991, 20, 271-339. It is known that the avermectins and milbemycins and their derivatives are active as antiparasitic agents.

Other avermectin and milbemycin derivatives which may be mentioned are disclosed in the following patents, applications, and their equivalents:
European - 214 731, 340 932, 334 848, 335 541, 350 187, 410 615 623 137, 712 411, 629 202, 702 688, 674 649, 710 242, 677 054 and 745 089;
WO 94/15944, and US 4,199,569.

The terms avermectins and milbemycins used herein includes both naturally occurring compounds and synthetic derivatives thereof, especially those mentioned in the art cited herein.

Bis-aryl compounds having insecticidal and acaricidal properties are also known. These compounds comprise directly joined aromatic rings which may include at least one nitrogen atom. Examples of this class of compounds include N-phenyl pyrazole derivatives having arthropodicidal, plant nematodicidal anthelmintic and anti-protozoal properties.

WO98/11780 describes compositions containing a 1-phenyl pyrazole derivative and a macrocyclic lactone endectoparasiticide.

Cutler, S. L. in the Journal of Small Animal Practice, (1998 Feb) 39 (2) 86-7., "Ectopic Psoroptes cuniculi infestation in a pet rabbit.", discusses treatment with ivermectin injection followed by fipronil application.

It has now been found that certain avermectins and milbemycins display unexpected synergy with members of the above-mentioned bis-aryl compounds with respect to their antiparasitic activity, allowing more effective control of parasites, especially those affecting livestock and companion animals. Examples of such parasites incude heartworm and various ectoparasites in companion animals. This is particularly unexpected as impairment of biological performance would be anticipated for such a combination, given the reported mode of action of avermectin and milbemycin compounds as agonists at the invertebrate glutamate gated chloride channel- receptor complex and the antagonist activity of the bis-aryl compounds at this site.

According to one aspect of the invention, there is provided a composition comprising a combination of a bis-aryl compound and an avermectin or milbemycin or derivative thereof, and if necessary a suitable pharmaceutical or veterinary carrier, wherein the bis-aryl compound is fipronil and the avermectin or milbemycin is selamectin. The invention also includes the use of such a composition in medicine, for instance for treating or preventing parasitic infestations in humans or animals, including infestation by heartworm and other parasites in companion animals such as dogs and cats.

Another aspect of the invention is the administration to an animal of a bis-aryl as defined above compound and an avermectin or milbemycin as defined above either in combination in the same composition, or via separate treatments, either substantially simultaneously or at spaced intervals, for treating or preventing a parasitic infestation. Different modes of administration can be envisaged by the skilled person where the bis-aryl compound is administered separately from the avermectin or milbemycin compound.

Another aspect to the invention is the use of a composition comprising an avermectin or milbemycin as defined above and a bis-aryl compound as defined above in the manufacture of an antiparasitic medicament.

Another aspect of the invention is a pharmaceutical pack comprising a bis-aryl compound as defined above and an avermectin or milbemycin as defined above.

For use in mammals, including humans, the compounds, either alone or in the combinations mentioned above, can be administered alone, but will generally be administered in admixture with a pharmaceutically or veterinarily acceptable diluent or carrier selected with regard to the intended route of administration and standard pharmaceutical practice. For example, they can be administered orally, including sublingually, in the form of tablets containing such excipients as starch or lactose, or in capsules or ovules either alone or in admixture with excipients, or in the form of elixirs, solutions or suspensions containing flavouring or colouring agents. The compounds could be incorporated into capsules or tablets for targetting the colon or duodenum via delayed dissolution of said capsules or tablets for a particular time following oral administration. Dissolution could be controlled by susceptibility of the formulation to bacteria found in the duodenum or colon, so that no substantial dissolution takes places before reaching the target area of the gastrointestinal tract. The compounds can be injected parenterally, for example, intravenously, intramuscularly or subcutaneously. For parenteral administration, they are best used in the form of a sterile aqueous solution or suspension which may contain other substances, for example, enough salt or glucose to make the solution isotonic with blood. They can be administered topically, in the form of sterile creams, gels, suspensions, lotions, ointments, dusting powders, sprays, drug-incorporated dressings or via a skin patch. For example they can be incorporated into a cream consisting of an aqueous or oily emulsion of polyethylene glycols or liquid paraffin, or they can be incorporated into an ointment consisting of a white wax soft paraffin base, or as hydrogel with cellulose or polyacrylate derivatives or other viscosity modifiers, or as a dry powder or liquid spray or aerosol with butane/propane, HFA or CFC propellants, or as a drug-incorporated dressing either as a tulle dressing, with white soft paraffin or polyethylene glycols impregnated gauze dressings or with hydrogel, hydrocolloid, alginate or film dressings. The compounds could also be administered intraocularly as an eye drop with appropriate buffers, viscosity modifiers (e.g. cellulose derivatives), preservatives (e.g. benzalkonium chloride (BZK)) and agents to adjust tenicity (e.g. sodium chloride). Such formulation techniques are well-known in the art. In some instances the formulations may advantageously also contain an antibiotic. All such formulations may also contain appropriate stabilisers and preservatives.

For veterinary use, compounds can be administered as a suitably acceptable formulation in accordance with normal veterinary practice and the veterinary surgeon will determine the dosing regimen and route of administration which will be most appropriate for a particular animal.

For topical application dip, spray, powder, dust, pour-on, spot-on, emulsifiable concentrate, jetting fluid, shampoos, collar, tag or harness may be used. Such formulations are prepared in a conventional manner in accordance with standard veterinary and pharmaceutical practice. Thus capsules, boluses or tablets may be prepared by mixing the active ingredient with a suitable finely divided diluent or carrier, additionally containing a disintegrating agent and/or binder such as starch, lactose, talc, or magnesium stearate. A drench formulation may be prepared by dispersing the active ingredients in an aqueous solution together with dispersing or wetting agents and injectable formulations may be prepared in the form of a sterile solution or emulsion. Pour-on or spot-on formulations may be prepared by dissolving the active ingredients in an acceptable liquid carrier vehicle, such as butyl digol, liquid paraffin or non-volatile ester with or without addition of a volatile component such as isopropanol.

Alternatively, pour-on, spot-on or spray formulations can be prepared by encapsulation to leave a residue of active agent on the surface of the animal. These formulations will vary with regard to the weight of active compound depending on the species of host animal to be treated, the severity and type of infection and type and body weight of the host. The combinations may be administered continuously, particularly for prophylaxis by known methods. Generally for oral, parenteral and pour-on administration a dose of from about 0.001 to 10mg per kg of animal body weight given as a single dose or in divided doses for a period of from 1 to 5 days will be satisfactory but of course there can be instances where higher or lower dosage ranges are indicated and such are within the scope of this invention.

As an alternative the combinations may be administered with the animal feedstuff and for this purpose a concentrated feed additive or premix may be prepared for mixing with the normal animal feed.

For use as an insecticide and for treating agricultural pests the compounds are applied as sprays, dusts, pour-on formulations, emulsions and the like in accordance with standard agricultural practice.

For human use the combinations are administered as a pharmaceutically acceptable formulation in accordance with normal medical practice.

The combinations of compounds of the invention may be formulated as described above as a mixture, alternatively the avermectin or milbemycin compound and the bis-aryl compound may be administered as separate doses and such treatment is still within the scope of the invention.

The compositions, treatments, etc, of the invention may be combined with other agents, treatments, etc. useful against certain other diseases or in the reduction or suppression of other symptoms. Examples of such agents (which are provided by way of illustration and should not be construed as limiting) include other antiparasitics, eg lufenuron, imidacloprid, organophosphates, pyrethroids; antihistamines, eg chlorpheniramine, trimeprazine, diphenhydramine, doxylamine; antifungals, eg fluconazole, ketoconazole, itraconazole, griseofulvin, amphotericin B; antibacterials, eg enroflaxacin, marbofloxacin, ampicillin, amoxycillin; anti-inflammatories eg prednisolone, betamethasone, dexamethasone, carprofen, ketoprofen; dietary supplements, eg gamma-linoleic acid; and emollients. Therefore, the invention further provides a product containing a compound of the invention and one or more selected compounds from the above list as a combined preparation for simultaneous, separate or sequential use in the treatment of conditions mediated by parasites, and the related methods of treatment, etc.

The avermectin or milbemycin is selamectin described in International Patent Application PCT/EP94/00095.

The bis-aryl compound is 5-amino-3-cyano-1-(2,6-dichloro-4-trifluoromethylphenyl)-4-trifluoromethylsulphinylpyrazole), a compound known as fipronil described in EP-295117.

When used as pesticidal agent in a host animal the compositions of the invention may be applied to the human or animal host patient at a typical amount of 0.001-10 mgs/kg of body weight of avermectin or milbemycin compound. The weight ratio of avermectin or milbemycin compound to the aryl pyrazole will generally be in the range of 1 to 100, preferably 1 to 10. The synergistic effects of certain combinations allow a reduced dosage of both compounds to be employed to obtain a given antiparasitic effect, reducing risks of unwanted side effects, toxicity and development of resistance to the compounds concerned and longer duration of action.

The combination therapy of the invention is effective in treating a variety of conditions caused by endoparasites including, in particular, helminthiasis which is most frequently caused by a group of parasitic worms described as nematodes and which can cause severe economic losses in swine, sheep, horses and cattle as well as affecting domestic animals and poultry. The most common genera of nematodes infecting the animals referred to above are Haemonchus, Trichostrongylus, Ostertagia, Teladorsagia, Nematodirus, Cooperia, Ascaris, Bunostomum, Oesophagostomum, Chabertia, Strongylus, Trichonema and Dictyocaulus. The combinations are also effective against other nematodes which affect various species of animals including, for example:- Dirofilaria in dogs and various parasites which can infest livestock, companion animals such as cats and dogs and also humans including gastrointestinal parasites such as Ancylostoma, Uncinaria, Necator, Ascaris, Strongyloides, Trichinella, Capillaria, Toxocara, Toxascaris, Trichuris, Enterobius and parasites which are found in the blood or other tissues and organs such as filarial worms and the extra intestinal stages of Strongyloides, Toxocara and Trichinella. They are especially useful in treating heartworm in companion animals.

The combinations are also of value in treating ectoparasite infections including particular arthropod ectoparasites of host humans, animals and birds such as ticks, mites, lice, fleas, blowfly, biting insects and migrating dipterous larvae which can affect cattle and horses. The combinations are also insecticides active against household pests such as the cockroach, clothes moth, carpet beetle and the housefly as well as being useful against arthropod pests of stored grain and of agricultural plants such as spider mites, aphids, caterpillars and against migratory orthopterans such as locusts.

The efficacy of compositions according to an embodiment of the invention against heartworm (Dirofilaria immitis) is demonstrated by the following Example.

### Example

Infective L3 larvae of Dirofilaria immitis were recovered from previously infected Aedes egypti and cultured in vitro to the L4 stage by techniques described by D. Abraham et al (J. Parasit. 73(2) 1987, pp 377-383).

### Test procedure

Testing of compounds took place when >95% of the larvae had moulted to the L4 stage. The assay system consisted of a 96 well microtitre plate in which 79 mcl of assay media, 1 mcl test compound and 20 mcl assay media containing 15 to 20 L4 D. immitis were dispensed. Each test compound was dissolved in dimethylsulphoxide (DMSO) and dilutions were made using this solvent. The test microtitre plates were kept at 37 degrees centigrade under an atmosphere comprising 5% CO, in air for 72 h.

### Assay procedure

The effects of each compound at each concentration and combination was assessed by comparing the motility of L4 larvae in control and treated wells. Microscopic observations were made at 2, 4, 24, 48 and 72 hours on each well. Observations were scored according to the levels of larval motility, ranging from 0 (all dead) to 5 (all normal motility).

The above procedure was carried out using solutions containing the compounds shown in Table 1 below at the stated concentrations. Compound (1) is 5-oximino 22,23-dihydro-25-cyclohexylavermectin B1 monosaccharide (selamectin), disclosed as Example 5 in WO 94/15944.

**Table 1 In vitro L4 Heartworm (D.immitis)**

| Duplicate Observations (hours post treatment) | | | | | | |
|---|---|---|---|---|---|---|
| Compound/s | mcg/ml | 2h | 4h | 24h | 48h | 72h |
| CONTROL | - | 5/5 | 5/5 | 5/5 | 5/5 | 5/5 |
| (1) | 10 | 2/2 | 2/0 | 3/3 | 4/4 | 3/4 |
| (1) | 1 | 5/5 | 5/5 | 5/5 | 5/5 | 5/5 |
| Fipronil | 10 | 5/5 | 5/5 | 5/5 | 5/5 | 5/5 |
| (1) + Fipronil | 10 10 | 4/2 | 1/0 | 0/0 | 0/0 | 0/0 |
| (1)+ Fipronil | 10 1 | 4/4 | 1/3 | 1/0 | 2/3 | 0/3 |

| | | | | | | |
|---|---|---|---|---|---|---|
| Key - Observation Score: 0 = No motility, larvae dead 1= Motility in at least 1 larva, all others dead 2 =≥80% of larvae dead, others sluggish 3 = < 80% of larvae dead, others sluggish 4 = All larvae (or most) motile but sluggish 5 = Normal in appearance and motility. | | | | | | |

It can be seen from the results given in Table 1 that, whereas compound (1) alone had only a modest effect in causing mortality or paralysis and fipronil alone had no observable effect at all at concentrations of 10 mcg/ml, the combination of these compounds was very effective.

All publications mentioned above are herein incorporated by reference in their entirety.

Reference to treatment herein includes preventative, palliative and curative treatments.

## Claims

1. A composition comprising a combination of a bis-aryl compound and an avermectin or milbemycin or derivative thereof, and if necessary a suitable pharmaceutical or veterinary carrier, wherein the bis-aryl compound is fipronil and the avermectin or milbemycin is selamectin.

2. A composition according to claim 1 for use in medicine.

3. The use of an avermectin or milbemycin and a bis-aryl compound according to claim 1 in the manufacture of an antiparasitic medicament.

4. The use according to claim 3 wherein the avermectin or milbemycin and bis-aryl compound are administered either in combination in the same composition, or via separate treatments, either substantially simultaneously or at spaced intervals.

5. A pharmaceutical pack comprising an avermectin or milbemycin and a bis-aryl compound according to claim 1.

6. A method of killing or harming a parasite at a locus comprising administering to said locus an effective combination of an avermectin or milbemycin and a bis-aryl compound according to claim 1, wherein the locus is not on or in a human or non-human animal.

7. A process for making a composition according to claim 1 which comprises mixing a bis-aryl compound according to claim 1 with an avermectin or milbemycin according to claim 1, and optionally a pharmaceutical or veterinary carrier.

## Patentansprüche

1. Zusammensetzung, umfassend eine Kombination aus einer Bisaryl-Verbindung und einem Avermectin oder Milbemycin oder einem Derivat davon und, falls erforderlich, einem geeigneten pharmazeutischen oder veterinärmedizinischen Träger, wobei die Bisaryl-Verbindung Fipronil ist und das Avermectin oder Milbemycin Selamectin ist.

2. Zusammensetzung gemäß Anspruch 1 zur Verwendung in Arzneimitteln.

3. Verwendung eines Avermectins oder Milbemycins und einer Bisaryl-Verbindung gemäß Anspruch 1 in der Herstellung eines antiparasitischen Medikaments.

4. Verwendung gemäß Anspruch 3, wobei das Avermectin oder Milbemycin und die Bisaryl-Verbindung entweder in Kombination in derselben Zusammensetzung oder über getrennte Behandlungen, entweder im Wesentlichen simultan oder in Intervallen mit Abstand, verabreicht werden.

5. Pharmazeutische Packung, umfassend ein Avermectin oder Milbemycin und eine Bisaryl-Verbindung gemäß Anspruch 1.

6. Verfahren zum Abtöten oder Schädigen eines Parasiten an einer Stelle, umfassend das Verabreichen einer wirksamen Kombination aus einem Avermectin oder Milbemycin und einer Bisaryl-Verbindung gemäß Anspruch 1 an die Stelle, wobei die Stelle nicht auf oder in einem Menschen oder nichtmenschlichen Tier ist.

7. Verfahren zum Herstellen einer Zusammensetzung gemäß Anspruch 1, welches das Mischen einer Bisaryl-Verbindung gemäß Anspruch 1 mit einem Avermectin oder Milbemycin gemäß Anspruch 1 und gegebenenfalls einem pharmazeutischen oder veterinärmedizinischen Träger umfasst.

## Revendications

1. Composition comprenant une combinaison d'un composé bis-aryle et d'une avermectine ou milbémycine ou d'un dérivé de celles-ci et, si nécessaire, un support convenable sur le plan pharmaceutique ou vétérinaire, où le composé bis-aryle est le fipronil et où l'avermectine ou milbémycine est la sélamectine.

2. Composition selon la revendication 1, pour une utilisation en médecine.

3. Utilisation d'une avermectine ou milbémycine et d'un composé bis-aryle selon la revendication 1, dans la fabrication d'un médicament antiparasitaire.

4. Utilisation selon la revendication 3, dans laquelle l'avermectine ou milbémycine et le composé bis-aryle sont administrés soit en combinaisons dans le même composition, soit via des traitements séparés, et soit sensiblement en même temps, soit à intervalles de temps espacés.

5. Présentation pharmaceutique comprenant une avermectine ou milbémycine et un composé bis-aryle selon la revendication 1.

6. Procédé de destruction ou de lésion d'un parasite en un site, comprenant l'administration audit site d'une combinaison efficace d'une avermectine ou milbémycine et d'un composé bis-aryle selon la revendication 1, le site n'étant ni sur, ni dans, un être humain ou un animal non-humain.

7. Procédé de préparation d'une composition selon la revendication 1, qui comprend le mélange d'un composé bis-aryle selon la revendication 1, avec une avermectine ou milbémycine selon la revendication 1 et, facultativement, un support convenable sur le plan pharmaceutique ou vétérinaire.
